# EUROPEAN PATENT APPLICATION

(11) **EP 4 631 929 A1**
(43) Date of publication of application: **15.10.2025**
(21) Application number: 23900643.0
(22) Date of filing: 05.12.2023
(51) Int. Cl.: C07C 209/72, B01J 23/46, B01J 23/96, B01J 37/18, B01J 38/10, B01J 38/48, C07C 211/18

(54) **METHOD FOR PRODUCING DIAMINE CONTAINING CYCLOHEXANE RING**

(30) Priority: 07.12.2022 JP 2022195714
(71) Applicant: MITSUBISHI GAS CHEMICAL COMPANY, INC., Chiyoda-ku Tokyo 100-8324 (JP)
(72) Inventor: SUGITA Takuya, Niigata-shi, Niigata 950-3121 (JP); YAMAICHI Aoto, Niigata-shi, Niigata 950-3121 (JP); MISAKI Satoshi, Niigata-shi, Niigata 950-3121 (JP); MUNEYASU Kuniaki, Niigata-shi, Niigata 950-3121 (JP)
(74) Representative: Müller-Boré & Partner Patentanwälte PartG mbB
(86) International application number: PCT/JP2023/043413
(87) International publication number: WO 2024/122528

(57) **Abstract**

A method for producing a cyclohexane ring-containing diamine, the method comprising hydrogenating an aromatic ring-containing diamine in the presence of a metal-containing alumina catalyst regenerated in a catalyst regeneration step and a solvent containing at least one selected from the group consisting of alkylamines and alkyldiamines, wherein the catalyst regeneration step includes a step (1) of cleaning the metal-containing alumina catalyst using water at 0 to 28°C; and a step (2) of bringing the metal-containing alumina catalyst into contact with a hydrogen-containing gas at more than 200°C and 500°C or less after the step (1).

## Description

### Technical Field

The present invention relates to a method for producing a cyclohexane ring-containing diamine and a method for regenerating a catalyst.

### Background Art

Cyclohexane ring-containing diamines are used for curing agents for epoxy resins, synthetic starting materials of organic compounds such as optically active substances, starting materials or intermediates for resins, and the like. Among these, bis(aminomethyl)cyclohexane is an industrially important compound as a starting material for bis(isocyanatomethyl)cyclohexane, or the like. Therefore, various studies have been performed on the method for producing a cyclohexane ring-containing diamine.

For example, PTL 1 describes a method for producing bis(aminomethyl)cyclohexane by catalytic hydrogenation using a ruthenium catalyst and a solvent selected from alkylamines and alkylenediamines.

In addition, PTL 2 discloses, as the method for producing bis(aminomethyl)cyclohexane, a method in which a catalyst having a reduced activity due to use is treated in a catalyst regeneration treatment step and then is reused in a reaction system, where the catalyst regeneration treatment step is a step of maintaining an amount of bis(aminomethyl)cyclohexane in a liquid at 20% by mass or less, heating a catalyst to 100 to 500°C, and bringing it into contact with a hydrogen-containing gas.

### Citation List

### Patent Literature

PTL1: JP 08-143514 A
PTL2: WO2021/131912

### Summary of Invention

### Technical Problem

In the production of cyclohexane ring-containing diamines, there is a problem that the catalytic activity gradually decreases with long-term use, failing to obtain a satisfactory yield. Therefore, attempts have been made to solve the above problems by, for example, the method described in PTL 2. Recently, in particular, from the viewpoint of load on environment or production efficiency, in the method for producing the diamine, such a method that enables extremely long-term continuous production without catalyst replacement has been required. According to the catalyst regeneration by the method disclosed in PTL 2, the activity of the catalyst can be recovered, but deposited products due to catalyst elution have not been still sufficiently suppressed. The deposited products cause malfunctions in heat exchangers and filters on the production line of the cyclohexane ring-containing diamines, causes hinderance of continuous operation, and causes inhibition of catalyst regeneration due to generation of deposited products in the catalyst. Furthermore, in order to decrease the frequency of catalyst replacement, it has been desired to recover the catalytic activity to an activity closer to that before use.

Therefore, an object of the present invention is to provide a method for producing a cyclohexane ring-containing diamine, which can efficiently recover catalytic activity while catalyst elution at the time of catalyst regeneration is suppressed and can produce a cyclohexane ring-containing diamine industrially and continuously.

### Solution to Problem

As a result of diligent studies, the present inventors have found that the problems can be solved by a method for regenerating a metal-containing alumina catalyst by a specific method, to hydrogenate an aromatic ring-containing diamine using the catalyst regenerated in the presence of a specific solvent.
The present invention relates to the following [1] to [8].
[1] A method for producing a cyclohexane ring-containing diamine, the method comprising hydrogenating an aromatic ring-containing diamine in the presence of a metal-containing alumina catalyst regenerated in a catalyst regeneration step comprising the following step (1) and step (2) and a solvent containing at least one selected from the group consisting of alkylamines and alkyldiamines:
   step (1): a step of cleaning the metal-containing alumina catalyst using water at 0 to 28°C; and
   step (2): a step of bringing the metal-containing alumina catalyst into contact with a hydrogen-containing gas at more than 200°C and 500°C or less after the step (1).
[2] The method for producing a cyclohexane ring-containing diamine according to [1], wherein pH of the water during the cleaning in the step (1) is 7 to 13.
[3] The method for producing a cyclohexane ring-containing diamine according to [1] or [2], wherein time for cleaning using water in the step (1) is 15 to 100 hours.
[4] The method for producing a cyclohexane ring-containing diamine according to any one of [1] to [3], wherein the metal-containing alumina catalyst is at least one selected from the group consisting of a ruthenium-containing alumina catalyst, a rhodium-containing alumina catalyst, a nickel-containing alumina catalyst, a palladium-containing alumina catalyst, and a platinum-containing alumina catalyst.
[5] The method for producing a cyclohexane ring-containing diamine according to any one of [1] to [4], wherein the aromatic ring-containing diamine is phenylenediamine, xylylenediamine, diaminodiphenylmethane, bis(aminomethyl)biphenyl, or benzidine.
[6] The method for producing a cyclohexane ring-containing diamine according to any one of [1] to [5], wherein the cyclohexane ring-containing diamine is diaminocyclohexane, bis(aminomethyl)cyclohexane, methylenebis(cyclohexylamine), bicyclohexanediyldimethanamine, or bicyclohexanediamine.
[7] The method for producing a cyclohexane ring-containing diamine according to any one of [1] to [6], wherein the solvent containing at least one selected from the group consisting of alkylamines and alkyldiamines is at least one selected from the group consisting of methylamine, dimethylamine, trimethylamine, ethylamine, diethylamine, triethylamine, propylamine, dipropylamine, isopropylamine, diisopropylamine, butylamine, dibutylamine, tributylamine, hexylamine, cyclohexylamine, 2-ethylhexylamine, ethylenediamine, propylenediamine, 1,4-butylenediamine, hexamethylenediamine, and bis(aminomethyl)cyclohexane.
[8] A method for regenerating a catalyst, the method comprising hydrogenating an aromatic ring-containing diamine in the presence of a metal-containing alumina catalyst, and a solvent containing at least one selected from the group consisting of alkylamines and alkyldiamines, to obtain a cyclohexane ring-containing diamine, and then treating the metal-containing alumina catalyst in a regeneration treatment step comprising the following step (1) and step (2):
   step (1): a step of cleaning the metal-containing alumina catalyst using water at 0 to 28°C; and
   step (2): a step of bringing the metal-containing alumina catalyst into contact with a hydrogen-containing gas at more than 200°C and 500°C or less after the step (1).

### Advantageous Effects of Invention

According to the production method of the present invention, it is possible to provide a method for producing a cyclohexane ring-containing diamine, which suppresses catalyst elution at the time of catalyst regeneration, can efficiently recover the catalytic activity, and can produce the cyclohexane ring-containing diamine industrially and continuously. In addition, according to the method for regenerating a catalyst of the present invention, it is possible to efficiently recover the catalytic activity of the catalyst used for producing the cyclohexane ring-containing diamine while the catalyst elution at the time of catalyst regeneration is suppressed.

### Description of Embodiment

A method for producing a cyclohexane ring-containing diamine of the present invention is a method for producing a cyclohexane ring-containing diamine, the method comprising hydrogenating an aromatic ring-containing diamine in the presence of a metal-containing alumina catalyst regenerated in a catalyst regeneration step comprising the following step (1) and step (2) and a solvent containing at least one selected from the group consisting of alkylamines and alkyldiamines:
step (1): a step of cleaning the metal-containing alumina catalyst using water at 0 to 28°C; and
step (2): a step of bringing the metal-containing alumina catalyst into contact with a hydrogen-containing gas at more than 200°C and 500°C or less after the step (1).

According to the present invention, it is possible to efficiently recover catalytic activity while catalyst elution at the time of catalyst regeneration is suppressed, and to produce a cyclohexane ring-containing diamine industrially and continuously.

A mode for carrying out the present invention (hereinafter simply referred to as "this embodiment") will be described in detail below. This embodiment is an illustration for describing the present invention and is not intended to limit the present invention to the following contents. Appropriate modifications can be made to the present invention without departing from the spirit thereof. As used herein, the description "XX to YY" means "XX or more and YY or less".

### [Method for Producing Cyclohexane Ring-Containing Diamine and Method for Regenerating Catalyst]

A method for producing a cyclohexane ring-containing diamine of the present invention is a method for producing a cyclohexane ring-containing diamine, the method comprising hydrogenating an aromatic ring-containing diamine in the presence of a metal-containing alumina catalyst regenerated in a catalyst regeneration step comprising the following step (1) and step (2) and a solvent containing at least one selected from the group consisting of alkylamines and alkyldiamines.

In addition, a method for regenerating a catalyst of the present invention is a method for regenerating a catalyst, the method comprising hydrogenating an aromatic ring-containing diamine in the presence of a metal-containing alumina catalyst, and a solvent containing at least one selected from the group consisting of alkylamines and alkyldiamines, to obtain a cyclohexane ring-containing diamine, and then treating the metal-containing alumina catalyst in a regeneration treatment step comprising the following step (1) and step (2):
step (1): a step of cleaning the metal-containing alumina catalyst using water at 0 to 28°C; and
step (2): a step of bringing the metal-containing alumina catalyst into contact with a hydrogen-containing gas at more than 200°C and 500°C or less after the step (1).

That is, in the method for producing a cyclohexane ring-containing diamine of the present invention, the metal-containing alumina catalyst used for the hydrogenation is the catalyst regenerated by the regeneration method of the catalyst. Therefore, in the method for producing a cyclohexane ring-containing diamine of the present invention, the metal-containing alumina catalyst used for the hydrogenation is a catalyst regenerated in the catalyst regeneration step including the step (1) and the step (2).

The step (1) and the step (2) in the method for producing the cyclohexane ring-containing diamine of the present invention and the step (1) and the step (2) in the method for regenerating the catalyst of the present invention are the same steps, and the hydrogenation in the method for regenerating a catalyst of the present invention and the hydrogenation in the method for producing the cyclohexane ring-containing diamine of the present invention are the same. Hereinafter, regarding the method for producing a cyclohexane ring-containing diamine of the present invention, each step and hydrogenation will be described, but preferable embodiments of each step and hydrogenation are the same as preferable embodiments of each step and hydrogenation in the method for regenerating a catalyst of the present invention. That is, the following description is the description of the method for producing a cyclohexane ring-containing diamine of the present invention, and is also the description of the method for regenerating a catalyst of the present invention.

### [Step (1) (Cleaning Step)]

The method for producing a cyclohexane ring-containing diamine of the present invention uses a metal-containing alumina catalyst regenerated in a catalyst regeneration step that includes the step (1) and the step (2), and the step (1) is a step of cleaning the metal-containing alumina catalyst using water at 0 to 28°C.

### <Metal-Containing Alumina Catalyst>

In the method for producing a cyclohexane ring-containing diamine of the present invention, the metal-containing alumina catalyst used for the hydrogenation is a catalyst regenerated in the catalyst regeneration step that includes the step (1) and the step (2). Therefore, the metal-containing alumina catalyst subjected to this step is a catalyst used for hydrogenation one or more times, and the step (1) is a step of cleaning the metal-containing alumina catalyst used for hydrogenation one or more times using water at 0 to 28°C.

The metal-containing alumina catalyst is preferably at least one selected from the group consisting of a precious metal-containing alumina catalyst and a nickel-containing alumina catalyst, and more preferably a precious metal-containing alumina catalyst. More specifically, the metal-containing alumina catalyst is at least one selected from the group consisting of a ruthenium-containing alumina catalyst, a rhodium-containing alumina catalyst, a nickel-containing alumina catalyst, a palladium-containing alumina catalyst, and a platinum-containing alumina catalyst, more preferably at least one selected from the group consisting of a ruthenium-containing alumina catalyst, a rhodium-containing alumina catalyst, a palladium-containing alumina catalyst, and a platinum-containing alumina catalyst, and still more preferably a ruthenium-containing alumina catalyst.

The metal-containing alumina catalyst is preferably such a form that the metal is supported on alumina, and preferably a metal-supported alumina catalyst.

The amount of the metal of the metal-supported alumina catalyst supported may be appropriately selected depending on, for example, the form of the catalyst, the type of the metal, the temperature of the hydrogenation reaction, and the amount of hydrogen supplied. The amount of the ruthenium-supported alumina catalyst supported, in which ruthenium is supported on alumina, is preferably a catalyst obtained by supporting ruthenium on alumina in an amount of 1.5 to 2.5% by mass. The form is preferably a catalyst obtained by supporting ruthenium on 1 mm ϕ to 2 mm ϕ crushed grain alumina.

### <Cleaning Conditions>

In the step (1), the metal-containing alumina catalyst is cleaned using water at 0 to 28°C.

The catalyst before cleaning still contains residues from the hydrogenation reaction (reaction in which a cyclohexane ring-containing diamine is obtained by hydrogenating an aromatic ring-containing diamine) performed before step (1). The residues contain various compounds, such as a cyclohexane ring-containing diamine, which is a product of the hydrogenation reaction, a solvent, a starting material, and a by-product.

Cleaning in this step makes it possible to efficiently remove these residues, suppress the catalyst elution, and recover the catalytic activity.

In this step, a method for cleaning the catalyst is not particularly limited as long as it is a method for cleaning the catalyst using water at 0 to 28°C. It may be a method for cleaning the catalyst in a reactor used for the hydrogenation reaction, or may be a method in which the catalyst is extracted from a reactor and is cleaned in another container. However, a method for cleaning the catalyst in a reactor is preferable because it can simplify the process and can be industrially advantageous.

As the reactor, either a fixed-bed reactor or a slurry bed reactor may be used, but a fixed-bed reactor is preferable. In addition, either a batch-type reactor or a continuous-type reactor may be used, but a continuous-type reactor is preferable. Therefore, a fixed-bed continuous flow type reactor is more preferable.

The water used for cleaning may contain a water-soluble organic solvent within a range that does not impair the effects of the present invention. The water-soluble organic solvent is preferably alcohols. Examples of the alcohols include methanol, ethanol, isopropyl alcohol, and n-propyl alcohol. From the viewpoint of cost and cleaning efficiency, the water used for cleaning preferably does not substantially contain an organic solvent other than water, more preferably does not contain an organic solvent other than water, and still more preferably is only water.

The specific cleaning method is not limited, but a method in which water is allowed to flow through the catalyst is preferable, and a method in which water is allowed to flow through the catalyst while the water is circulated is more preferable. Allowing water to flow makes it possible to efficiently remove the residues by water flow.

The cleaning temperature is 0 to 28°C, preferably 3 to 28°C, more preferably 10 to 28°C, still more preferably 15 to 25°C, even still more preferably 18 to 25°C, and even still more preferably 20 to 25°C. When the cleaning temperature falls within the above range, it is possible to significantly decrease the catalyst elution and to recover the catalytic activity. The cleaning temperature is preferably a low temperature, but when it is 28°C or less, the catalyst can be favorably regenerated. Therefore, from the viewpoint of efficiently recovering the catalytic activity while the consumption of energy required for cooling water is suppressed, the cleaning temperature is preferably 3 to 28°C, more preferably 10 to 28°C, still more preferably 15 to 28°C, even still more preferably 18 to 28°C, even still more preferably 20 to 28°C, even still more preferably 22 to 28°C, even still more preferably 23 to 28°C, and even still more preferably 23 to 27°C.

The time for cleaning using water (cleaning time) is preferably 15 to 300 hours, more preferably 20 to 250 hours, still more preferably 30 to 100 hours, even still more preferably 30 to 80 hours, even still more preferably 35 to 75 hours, even still more preferably 35 to 70 hours, even still more preferably 35 to 65 hours, even still more preferably 40 to 65 hours, and even still more preferably 40 to 60 hours. The "time for cleaning using water (cleaning time)" refers to the time from the start of flowing water, to the final removal of the water by solid-liquid separation via circulation and water exchange. When the cleaning time falls within the above range, it is possible to significantly decrease the catalyst elution and to recover the catalytic activity.

In the step (1), the pH of the water during cleaning is preferably 7 to 13.5, more preferably 7 to 13.2, and still more preferably 7 to 13. From the viewpoint of suppressing the amount of water used, it is preferable to stop discharging water and to circulate the water at the time when the pH of the water during cleaning reaches 13.5 or less. From the viewpoint of more efficiently recover the catalytic activity, it is more preferable to stop discharging water and to circulate the water at the time when the pH of the water during cleaning reaches 13.2 or less, and it is still more preferable to stop discharging water and to circulate the water at the time when the pH of the water during cleaning reaches 13 or less. When the pH of water falls within the above range, it is possible to significantly decrease the catalyst elution and to recover the catalytic activity. Note that, when the pH at the time when water starts to flow does not fall within the above range, the catalyst may be immersed in water to remove large amounts of residues by solid-liquid separation before the water is allowed to flow, or a neutralizer for adjusting the pH may be added.

After the completion of this step, the water (aqueous solution) after cleaning is removed. The removal method is preferably solid-liquid separation, and when this step is performed in a reactor, the water (aqueous solution) after cleaning may be discharged from the bottom of the reactor. In order to dry the catalyst, heating, decompression, or flowing of a dry gas may be performed.

### [Step (2) (Hydrogen Contact Step)]

The method for producing a cyclohexane ring-containing diamine of the present invention is a production method using the metal-containing alumina catalyst regenerated in the catalyst regeneration step that includes the step (1) and the step (2). The step (2) is a step of bringing the metal-containing alumina catalyst into contact with a hydrogen-containing gas at more than 200°C and 500°C or less after the step (1).

The hydrogen-containing gas used in this step may contain an inert gas that does not interfere with catalyst regeneration as long as it contains hydrogen gas. Examples of the inert gas include methane and nitrogen. The concentration of the hydrogen gas contained in the hydrogen-containing gas is preferably 50 to 100% by volume, more preferably 80 to 100% by volume, and still more preferably 90 to 100% by volume. The hydrogen-containing gas is still more preferably hydrogen gas.

The temperature at which the catalyst is brought into contact with the hydrogen-containing gas in this step is more than 200°C and 500°C or less, preferably 210 to 400°C, more preferably 220°C to 350°C, more preferably 220°C to 300°C, more preferably 220°C to 290°C, and more preferably 240°C to 290°C. In this step, the temperature during the treatment may be changed within the above range. For example, a constant temperature holding process may be combined with a temperature rise or temperature fall process. When the temperature at which the catalyst is brought into contact with the hydrogen-containing gas falls within the above range, the catalyst can be prevented from being deteriorated, and the catalytic activity can be recovered. Note that, the "more than 200°C and 500°C or less" means a temperature that is greater than 200°C and is 500°C or less.

The hydrogen-containing gas supply rate in this step may be appropriately adjusted depending on the content of hydrogen in the hydrogen-containing gas, the type of catalyst, the temperature, and the like, but is preferably 0.001 to 2000 NL/hour (N: standard state (0°C, 1 atm)), more preferably 0.1 to 1000 NL/hour, still more preferably 10 to 500 NL/hour, even still more preferably 100 to 400 NL/hour, and even still more preferably 200 to 300 NL/hour, per 1 kg of catalyst.

The time for bringing the catalyst into contact with the hydrogen-containing gas is preferably 3 hours or more. When the degree of activity decrease is severe, time for bringing the catalyst into contact with the hydrogen-containing gas is preferably extended. The time for bringing the catalyst into contact with the hydrogen-containing gas is more preferably 3 to 300 hours, preferably 5 to 100 hours, more preferably 7 to 30 hours, still more preferably 7 to 20 hours, and even still more preferably 7 to 15 hours. When the time for bringing the catalyst into contact with the hydrogen-containing gas falls within the above range, the catalytic activity can be efficiently recovered.

The hydrogen pressure in this step is preferably 0.01 kPa to 30 MPa, more preferably 0.1 kPa to 15 MPa, still more preferably 1 kPa to 10 MPa, even still more preferably 10 kPa to 5 MPa, even still more preferably 50 kPa to 1 MPa, and more preferably 50 kPa to 0.5 MPa. When the hydrogen-containing gas contains gas other than hydrogen, the hydrogen pressure refers to a partial pressure of the hydrogen gas.

In this step, as long as the metal-containing alumina catalyst can be brought into contact with the hydrogen-containing gas at more than 200°C and 500°C or less, a container to be used is not limited, but the metal-containing alumina catalyst is preferably brought into contact with the hydrogen-containing gas in the container used in the step (1). In addition, the metal-containing alumina catalyst is preferably brought into contact with the hydrogen-containing gas in the reactor for producing the cyclohexane ring-containing diamine. As described above, when the step (1) and the step (2) are performed in the reactor for producing the cyclohexane ring-containing diamine, the process can be simplified, which is industrially advantageous. Therefore, the step (1) and the step (2) are preferably performed in the reactor.

### [Hydrogenation of Aromatic Ring-Containing Diamine]

The method for producing a cyclohexane ring-containing diamine of the present invention is a method for hydrogenating an aromatic ring-containing diamine in the presence of a metal-containing alumina catalyst regenerated in a catalyst regeneration step comprising the step (1) and the step (2) and a solvent containing at least one selected from the group consisting of alkylamines and alkyldiamines.

### <Solvent>

This hydrogenation reaction is performed in the presence of a solvent containing at least one selected from the group consisting of alkylamines and alkyldiamines.

The solvent is preferably liquid at room temperature (25°C).

The solvent is a solvent containing at least one selected from the group consisting of alkylamines and alkyldiamines, and preferably a solvent containing at least alkyldiamines.

The solvent is preferably at least one selected from the group consisting of alkylamines and alkyldiamines, and more preferably alkyldiamines.

The solvent is preferably a solvent containing at least one selected from the group consisting of alkylamines having 1 to 18 carbon atoms and alkylenediamines having 1 to 18 carbon atoms, and more preferably a solvent containing at least alkyldiamines having 1 to 18 carbon atoms.

The solvent containing at least one selected from the group consisting of alkylamines and alkyldiamines is preferably at least one selected from the group consisting of methylamine, dimethylamine, trimethylamine, ethylamine, diethylamine, triethylamine, propylamine, dipropylamine, isopropylamine, diisopropylamine, butylamine, dibutylamine, tributylamine, hexylamine, cyclohexylamine, 2-ethylhexylamine, ethylenediamine, propylenediamine, 1,4-butylenediamine, hexamethylenediamine, and bis(aminomethyl)cyclohexane.

Among the above solvents, the cyclohexane ring-containing diamine, which is a product of the present invention, is preferably used as a solvent. Use of the cyclohexane ring-containing diamine, which is a product of the present invention, as a solvent is advantageous because separation from the product is not necessary.

From the viewpoint of the above, the solvent containing at least one selected from the group consisting of alkylamines and alkyldiamines is preferably a cyclohexane ring-containing diamine, is at least one selected from the group consisting of diaminocyclohexane, bis(aminomethyl)cyclohexane, methylenebis(cyclohexylamine), bicyclohexanediyldimethanamine, and bicyclohexanediamine, and is more preferably bis(aminomethyl)cyclohexane.

The solvent is a solvent containing at least one selected from the group consisting of alkylamines and alkyldiamines, but a mixture with another organic solvent can also be used.

Examples of another organic solvent include alcohols such as methanol, ethanol, isopropyl alcohol, and n-propyl alcohol.

The mass ratio (diamine:solvent) of the aromatic ring-containing diamine as the starting material in this hydrogenation reaction to the solvent is preferably 1:100 to 1:1, more preferably 1:80 to 1:3, and still more preferably 1:50 to 1:10. When a mixed solvent is used as the solvent, the total amount of the mixed solvent is preferably the above mass ratio.

### <Aromatic Ring-Containing Diamine>

The aromatic ring-containing diamine is a diamine corresponding to a desired cyclohexane ring-containing diamine. Therefore, it is a diamine obtained by replacing a cyclohexane ring of diamine of the desired product with an aromatic ring.

The aromatic ring-containing diamine used as the starting material in this hydrogenation reaction is preferably phenylenediamine, xylylenediamine, diaminodiphenylmethane, bis(aminomethyl)biphenyl, or benzidine, and more preferably xylylenediamine.

Hydrogenation of xylylenediamine makes it possible to obtain bis(aminomethyl)cyclohexane.

Xylylenediamine is preferably at least one selected from the group consisting of orthoxylylenediamine, metaxylylenediamine, and paraxylylenediamine, more preferably at least one selected from the group consisting of metaxylylenediamine and paraxylylenediamine, and still more preferably metaxylylenediamine. These may be used alone or in a mixture, but are preferably used alone.

### <Hydrogenation Conditions>

The hydrogenation of the aromatic ring-containing diamine is performed in the presence of the metal-containing alumina catalyst regenerated by the above step and the above solvent.

The form of the hydrogenation reaction may be either a fixed-bed form or a slurry bed form, but a fixed-bed form is preferable. In addition, either a batch-type or a continuous-type may be used, but a continuous-type is preferable. Therefore, a fixed-bed continuous flow type is more preferable because it is simple.

The hydrogen pressure in the hydrogenation of xylylenediamine is preferably 0.4 MPaG or more, and more preferably 5 to 14 MPaG for industrial purposes. The reaction temperature is preferably 50 to 150°C, and more preferably 80 to 130°C.

In the hydrogenation of xylylenediamine, when the reaction is performed in a continuous manner, the use amount of the metal-containing alumina catalyst regenerated in the catalyst regeneration step including the step (1) and the step (2) is preferably 0.001 to 5.0, and more preferably 0.01 to 1.0, in terms of WHSV relative to the amount of the aromatic ring-containing diamine of the starting material supplied.

More specifically, when an alumina catalyst, which supports approximately 2 mass% ruthenium, is used, the use amount thereof is preferably 0.001 to 5.0, and more preferably 0.01 to 1.0, in terms of WHSV relative to the amount of the aromatic ring-containing diamine of the starting material supplied.

In the hydrogenation reaction, an additive may be added for the purposes of reaction promotion, yield improvement, and the like. Examples of the additive include hydroxides and alcoholates of alkali metals or alkaline earth metals, and specific examples thereof include lithium hydroxide, sodium hydroxide, and potassium hydroxide.

In the hydrogenation reaction, the conversion rate of the aromatic ring-containing diamine is preferably 95 mol% or more, more preferably 98 mol% or more, still more preferably 98.9 mol% or more, even still more preferably 99 mol% or more, even still more preferably 99.5 mol% or more, and even still more preferably 99.8 mol% or more. A higher the conversion rate of the aromatic ring-containing diamine is preferable because the productivity is improved. The conversion rate decreases as the activity of the catalyst decreases. Therefore, in the case of continuous production, the catalyst regeneration step is preferably performed at the time when a predetermined conversion rate is reached. In actual production, for example, it is preferable to set the time when the conversion rate reaches 98.8 mol% to the timing for performing the catalyst regeneration step, and to perform the catalyst regeneration step after the hydrogenation reaction in which the conversion rate of the aromatic ring-containing diamine reaches 98.8 mol%.

When the hydrogenation reaction is performed in a continuous method, a gas-liquid separator is used to separate dissolved gas from a solution containing the product after the reaction, and a solvent is further removed by distillation to obtain a cyclohexane ring-containing diamine that is a target product.

Note that, when the cyclohexane ring-containing diamine that is a product is used as the solvent, the product may be used as a solvent for the next reaction. When the reaction is performed in a continuous method, a part of the product is collected and the remainder as the solvent may be returned to the reaction system.

### <Cyclohexane Ring-Containing Diamine>

The cyclohexane ring-containing diamine produced by the production method of the present invention is a diamine corresponding to the aromatic ring-containing diamine to be a starting material. Therefore, it is a diamine obtained by replacing the aromatic ring of the diamine to be a starting material with a cyclohexane ring.

The cyclohexane ring-containing diamine produced by the production method of the present invention is preferably diaminocyclohexane, bis(aminomethyl)cyclohexane, methylenebis(cyclohexylamine), bicyclohexanediyldimethanamine, or bicyclohexanediamine, and more preferably bis(aminomethyl)cyclohexane.

Hydrogenation of xylylenediamine makes it possible to obtain bis(aminomethyl)cyclohexane.

Bis(aminomethyl)cyclohexane is preferably at least one selected from the group consisting of 1,2-bis(aminomethyl)cyclohexane, 1,3-bis(aminomethyl)cyclohexane, and 1,4-bis(aminomethyl)cyclohexane, more preferably at least one selected from the group consisting of 1,3-bis(aminomethyl)cyclohexane and 1,4-bis(aminomethyl)cyclohexane, and still more preferably 1,3-bis(aminomethyl)cyclohexane. When the starting material is a mixture thereof, it can be obtained as a mixture, but is preferably obtained as a single compound.

### Examples

The present invention will be specifically described based on the Examples shown below, but the present invention is not limited by these Examples.

[Production of 1,3-Bis(Aminomethyl)Cyclohexane (Cyclohexane Ring-Containing Diamine)]

### Example 1

### <Hydrogenation of Metaxylylenediamine (Aromatic Ring-Containing Diamine) Using Commercially Available Catalyst>

The catalytic activity of a commercially available catalyst was confirmed, and the commercially available catalyst was used to hydrogenate metaxylylenediamine in order to obtain a catalyst after use, which would be subjected to the catalyst regeneration step.

As a catalyst, a commercially available 2% by mass ruthenium-supported alumina catalyst was used.

A fixed-bed continuous flow type tubular reactor (inner diameter 34 cm, filling height 200 cm) was filled with 130 kg of the catalyst, to form a catalyst layer. The catalyst was reduced and activated under a hydrogen gas flow at 260°C, and then a mixture including 5% by mass of metaxylylenediamine as a starting material and 95% by mass of 1,3-bis(aminomethyl)cyclohexane as a solvent was supplied to the reactor under the conditions of a reaction pressure of 8 MPaG and a reaction temperature of 100°C. The amount of the starting material supplied (referring to the mixture of the starting material and the solvent) at this time was 11 kg/h.

When the reaction liquid collected from the reactor exit was analyzed by gas chromatography, the conversion rate of metaxylylenediamine was 99.5 mol%. When the reaction was continued for 90 days under the same conditions, the conversion rate of metaxylylenediamine decreased to 98.8 mol%. At this time, the reaction was stopped.

The conversion rate of metaxylylenediamine (hereinafter also referred to as "MXDA") was calculated by the following method: Conversion rate (mol%) = ((MXDA mass in starting material supply liquid - MXDA mass in reactor exit liquid)/MXDA molecular weight)/(MXDA mass in starting material supply liquid/MXDA molecular weight) × 100

### <Catalyst Regeneration Step (1) (Step (1): Cleaning Step)>

After the pressure in the reactor was adjusted to normal pressure (atmospheric pressure), water was added thereto. Then, a liquid temperature was adjusted to 25°C, and water (cleaning liquid) was circulated while the liquid temperature was maintained at 25°C, to clean the catalyst layer used in the hydrogenation of the metaxylylenediamine. Note that, before circulation, charging and discharging water was repeated, and it was confirmed that the pH of the water (cleaning liquid) in the reactor was 13 or less. Then, water was circulated and the catalyst layer was cleaned. The cleaning time (time for circulating water) was 48 hours.

### <Catalyst Regeneration Step (2) (Step (2): Hydrogen Contact Step)>

Next, hydrogen gas was supplied to the above catalyst layer after cleaning at a rate of 240 NL/hour per kg of catalyst, and the temperature was increased from 25°C to 260°C. Hydrogen flow was maintained at 260°C for 10 hours under a hydrogen pressure of 0.4 MPa, and the temperature was then lowered to 25°C.

### <Hydrogenation of Metaxylylenediamine (Aromatic Ring-Containing Diamine) Using Regenerated Catalyst>

The catalyst regenerated by the catalyst regeneration step was used to perform the hydrogenation reaction of metaxylylenediamine.

The catalyst was reduced and activated under a hydrogen flow at 260°C, and then a mixture, which consisted of 5% by mass of metaxylylenediamine as a starting material and 95% by mass of 1,3-bis(aminomethyl)cyclohexane as a solvent, was supplied to the reactor under conditions of a reaction pressure of 8 MPaG and a reaction temperature of 100°C. At this time, an amount of the starting material (which refers to a mixture of the above starting material and the solvent) supplied was 11 kg/hour.

When the reaction liquid collected from the reactor exit was analyzed by gas chromatography, the conversion rate of metaxylylenediamine was 99.8 mol%. When the reaction was continued for 90 days under the same conditions, the conversion rate of metaxylylenediamine was 98.8 mol%, and the catalyst life was equivalent to that of an unused commercially available catalyst, and there was no decrease in the catalyst life.

Furthermore, during the hydrogenation reaction after completion of the catalyst regeneration step, no blockage due to deposition of aluminum hydroxide derived from the support alumina of the catalyst was observed.

### Comparative Example 1

### <Catalyst Regeneration Step (1) (Step (1): Cleaning Step)>

A commercially available catalyst was used to hydrogenate metaxylylenediamine in the same manner as in Example 1, and the reaction was continued for 90 days. Then, it was confirmed that the conversion rate of metaxylylenediamine decreased to 98.8 mol%, followed by stopping the reaction.

After the pressure in the reactor was adjusted to normal pressure (atmospheric pressure), water was added thereto. Then, a liquid temperature was adjusted to 70°C, and water (cleaning liquid) was circulated while the liquid temperature was maintained at 70°C, to clean a catalyst layer. Note that, before circulation, charging and discharging water was repeated, and it was confirmed that the pH of the water (cleaning liquid) in the reactor was 13 or less. Then, water was circulated and the catalyst layer was cleaned. The cleaning time (time for circulating water) was 48 hours.

### <Catalyst Regeneration Step (2) (Step (2): Hydrogen Contact Step)>

Next, hydrogen gas was supplied to the above catalyst layer after cleaning at a rate of 240 NL/hour per kg of catalyst, and the temperature was increased from 25°C to 260°C. Hydrogen flow was maintained at 260°C for 10 hours under a hydrogen pressure of 0.4 MPa, and the temperature was then lowered to 25°C.

### <Hydrogenation of Metaxylylenediamine (Aromatic Ring-Containing Diamine) Using Regenerated Catalyst>

The catalyst regenerated by the catalyst regeneration step was used to perform the hydrogenation reaction of metaxylylenediamine in the same manner as in Example 1. At the time when the catalyst was allowed to react for 24 hours, a pump strainer unit was blocked by aluminum hydroxide derived from the catalyst support, and the reaction could not be continued.

Then, the aluminum hydroxide that had clogged the pump strainer unit was removed, and the reaction was continued under the same conditions. After 55 days, the conversion rate of metaxylylenediamine decreased to 98.8 mol%.

As described above, it is found that when the catalysts of Examples that had experienced the catalyst regeneration step are used, blockage due to deposition of aluminum hydroxide derived from the support alumina of the catalyst is not caused, and the conversion rate of metaxylylenediamine is equivalent to that of a commercially available catalyst before use. Therefore, according to the production method of the present invention, it is found that the catalytic activity can be efficiently recovered while catalyst elution at the time of catalyst regeneration is suppressed, and a cyclohexane ring-containing diamine can be produced industrially and continuously.

### [Influence of Step (1) (Model Experiment)]

### Test Example 1

A model experiment was performed to confirm an influence of the step (1) (the cleaning step of the catalyst regeneration step) on a catalyst after use.

An autoclave having a volume of 20 mL was charged with 1.0 g of a catalyst (1), 2 g of 1,3-bis(aminomethyl)cyclohexane as a residual cyclohexane ring-containing diamine, and 8 g of water (diamine concentration 20%) as a cleaning liquid, and nitrogen gas was purged into the gas phase. A lid was then put thereon and the autoclave was maintained under the conditions (cleaning temperature and cleaning time) shown in Table 1. The catalyst was then extracted, cleaned with water, and dried at 60°C for 24 hours. The concentration of alumina hydrate in the dried catalyst was measured by the following method, and the appearance of the catalyst was evaluated based on the following criteria. The catalyst (1) used in this test is a 2% by mass ruthenium-supported alumina catalyst, which was used under the same conditions as in <Hydrogenation of Metaxylylenediamine (Aromatic Ring-Containing Diamine) Using Commercially Available Catalyst> of the Example 1, and exhibited a reduced conversion rate.

### <Alumina Hydrate Concentration>

The catalyst after drying was ground in an agate mortar until it was uniform, and X-ray diffraction analysis (measurement conditions: X-ray generator; 300 W/600 W sealing tube A, Cu (monochromatic Kβ filter), scanning range; 10 to 100 deg, step 0.02 deg, speed 10 deg/min) was performed using an X-ray diffraction device (MiniFlex600, Rigaku Corporation), and the concentrations of γ-alumina (Al₂O₃), boehmite (AlOOH, alumina monohydrate), and gibbsite (Al(OH)₃, aluminum hydroxide) were calculated using the RIR (reference intensity ratio) method. The total amount of boehmite and gibbsite relative to the total amount of γ-alumina, boehmite, and gibbsite was defined as the alumina hydrate concentration (mass%). A smaller value of the alumina hydrate concentration is preferable because the catalyst elution is further suppressed.

### <Catalyst Appearance after Cleaning>

The surface of the catalyst after drying was visually observed and evaluated based on the following criteria. The less the whitening of the surface of the catalyst, the less the catalyst is deteriorated, which is preferable.

### (Evaluation Criteria)

Good: No change is found in the surface of the catalyst (not whitened).
Fair: The surface of the catalyst is slightly whitened. The catalyst is not solidified at the bottom of the autoclave.
Poor: The surface of the catalyst is clearly whitened. The catalyst is solidified at the bottom of the autoclave.

### Test Examples 2 to 7 and Comparative Test Examples 1 to 4

Model experiments were performed in the same manner as in Test Example 1 except that the test conditions were changed as described in Table 1, and the extracted catalyst was evaluated in the same manner as in Test Example 1.

Specifically, Test Example 2 and Comparative Test Examples 1 to 3 were model experiments in the case where the cleaning temperature was changed. Test Example 3 was a model experiment in the case where the amount of diamine and pH were changed. Test Example 4 and Comparative Test Example 4 were model experiments in the case where the cleaning time was changed. Test Example 5 was a model experiment in the case where the diamine was changed. Test Examples 6 and 7 were model experiments in the case where the catalyst was changed. In addition, in the metal alumina catalysts in Table 1, "Ru/alumina" is a 2% by mass ruthenium-supported alumina catalyst, "Pd/alumina" is a 2% by mass palladium-supported alumina catalyst, and "Ni/alumina" is a 2% by mass nickel-supported alumina catalyst.

### [Table 1]

**Table 1**

| | Metal alumina catalyst | Diamine | Diamine concentration | Cleaning temperature | pH | Cleaning time | Catalyst appearance after cleaning | Alumina hydrate concentration |
|---|---|---|---|---|---|---|---|---|
| Test Example 1 | Ru/alumina | 1,3-Bis(aminomethyl)cyclohexane | 20% | 20°C | 12.6 | 72 hours | Good | 1.2% |
| Test Example 2 | Ru/alumina | 1,3-Bis(aminomethyl)cyclohexane | 20% | 4°C | 12.6 | 72 hours | Good | 1.0% |
| Comparative Test Example 1 | Ru/alumina | 1,3-Bis(aminomethyl)cyclohexane | 20% | 50°C | 12.6 | 72 hours | Fair | 5.9% |
| Comparative Test Example 2 | Ru/alumina | 1,3-Bis(aminomethyl)cyclohexane | 20% | 60°C | 12.6 | 72 hours | Poor | 7.2% |
| Comparative Test Example 3 | Ru/alumina | 1,3-Bis(aminomethyl)cyclohexane | 20% | 70°C | 12.6 | 72 hours | Poor | 8.9% |
| Test Example 3 | Ru/alumina | 1,3-Bis(aminomethyl)cyclohexane | 50% | 20°C | 13.5 | 72 hours | Good | 1.5% |
| Test Example 4 | Ru/alumina | 1,3-Bis(aminomethyl)cyclohexane | 20% | 20°C | 12.6 | 216 hours | Good | 1.3% |
| Comparative Test Example 4 | Ru/alumina | 1,3-Bis(aminomethyl)cyclohexane | 20% | 50°C | 12.6 | 216 hours | Fair | 6.9% |
| Test Example 5 | Ru/alumina | Diaminocyclohexane | 20% | 20°C | 13.2 | 72 hours | Good | 2.2% |
| Test Example 6 | Pd/alumina | 1,3-Bis(aminomethyl)cyclohexane | 20% | 20°C | 12.6 | 72 hours | Good | 2.1% |
| Test Example 7 | Ni/alumina | 1,3-Bis(aminomethyl)cyclohexane | 20% | 20°C | 12.6 | 72 hours | Good | 20% |

From the results in Table 1, it is found that the catalysts subjected to the step (1) in the production method of the present invention do not cause elution of aluminum hydroxide derived from the support alumina of the catalyst, and the catalysts are not deteriorated. According to this, it is found that the production method of the present invention makes it possible to efficiently recover catalytic activity while catalyst elution at the time of catalyst regeneration is suppressed. Therefore, it is found that a cyclohexane ring-containing diamine can be produced industrially and continuously according to the production method of the present invention.

## Claims

1. A method for producing a cyclohexane ring-containing diamine, the method comprising hydrogenating an aromatic ring-containing diamine in the presence of a metal-containing alumina catalyst regenerated in a catalyst regeneration step comprising the following step (1) and step (2) and a solvent containing at least one selected from the group consisting of alkylamines and alkyldiamines:
step (1): a step of cleaning the metal-containing alumina catalyst using water at 0 to 28°C; and
step (2): a step of bringing the metal-containing alumina catalyst into contact with a hydrogen-containing gas at more than 200°C and 500°C or less after the step (1).

2. The method for producing a cyclohexane ring-containing diamine according to claim 1, wherein pH of the water during the cleaning in the step (1) is 7 to 13.

3. The method for producing a cyclohexane ring-containing diamine according to claim 1 or 2, wherein time for cleaning using water in the step (1) is 15 to 100 hours.

4. The method for producing a cyclohexane ring-containing diamine according to any one of claims 1 to 3, wherein the metal-containing alumina catalyst is at least one selected from the group consisting of a ruthenium-containing alumina catalyst, a rhodium-containing alumina catalyst, a nickel-containing alumina catalyst, a palladium-containing alumina catalyst, and a platinum-containing alumina catalyst.

5. The method for producing a cyclohexane ring-containing diamine according to any one of claims 1 to 4, wherein the aromatic ring-containing diamine is phenylenediamine, xylylenediamine, diaminodiphenylmethane, bis(aminomethyl)biphenyl, or benzidine.

6. The method for producing a cyclohexane ring-containing diamine according to any one of claims 1 to 5, wherein the cyclohexane ring-containing diamine is diaminocyclohexane, bis(aminomethyl)cyclohexane, methylenebis(cyclohexylamine), bicyclohexanediyldimethanamine, or bicyclohexanediamine.

7. The method for producing a cyclohexane ring-containing diamine according to any one of claims 1 to 6, wherein the solvent containing at least one selected from the group consisting of alkylamines and alkyldiamines is at least one selected from the group consisting of methylamine, dimethylamine, trimethylamine, ethylamine, diethylamine, triethylamine, propylamine, dipropylamine, isopropylamine, diisopropylamine, butylamine, dibutylamine, tributylamine, hexylamine, cyclohexylamine, 2-ethylhexylamine, ethylenediamine, propylenediamine, 1,4-butylenediamine, hexamethylenediamine, and bis(aminomethyl)cyclohexane.

8. A method for regenerating a catalyst, the method comprising hydrogenating an aromatic ring-containing diamine in the presence of a metal-containing alumina catalyst, and a solvent containing at least one selected from the group consisting of alkylamines and alkyldiamines, to obtain a cyclohexane ring-containing diamine, and then treating the metal-containing alumina catalyst in a regeneration treatment step comprising the following step (1) and step (2):
step (1): a step of cleaning the metal-containing alumina catalyst using water at 0 to 28°C; and
step (2): a step of bringing the metal-containing alumina catalyst into contact with a hydrogen-containing gas at more than 200°C and 500°C or less after the step (1).
